# EUROPÄISCHE PATENTSCHRIFT

(11) **EP 1 382 316 B1**
(45) Veröffentlichungstag und Bekanntmachung des Hinweises auf die Patenterteilung: **17.08.2011**
(21) Anmeldenummer: 03014807.6
(22) Anmeldetag: 30.06.2003
(51) Int. Cl.: A61F 5/01

(54) **Handgelenksbandage**
Wrist bandage
Bandage pour poignet

(30) Priorität: 11.07.2002 DE 20211642 U
(43) Veröffentlichungstag der Anmeldung: 21.01.2004
(73) Patentinhaber: Ferd. Hauber GmbH, 72604 Nürtingen (DE)
(72) Erfinder: Röder, Daniel, 91074 Herzogenaurach / Niederndorf (DE)
(74) Vertreter: Dreiss

(56) Entgegenhaltungen:
- EP-A- 0 775 476
- WO-A-02/17827
- US-A- 4 854 309
- US-A- 5 160 314
- US-A- 5 713 837
- US-A- 5 749 841
- US-A- 6 102 880

## Beschreibung

Die vorliegende Erfindung bezieht sich auf eine Handgelenksbandage nach dem Oberbegriff des Anspruchs 1.

Bei derartigen derzeit im Handel befindlichen Handgelenksbandagen sind die beiden freien Enden des Textilteils an der Daumenseite einer Hand zusammengeführt, wobei das eine freie Ende mit einer Ausnehmung zur Aufnahme des Daumens aufweist. Der Verschluss besteht aus mehreren Riementeilen, von denen einer zwischen Daumen und Zeigefinger vom Handrücken zur Handinnenfläche geführt ist, während die anderen im Bereich des Unterarms umfangsseitig geführt sind.

Bei einer Handgelenksbandage nach dem Oberbegriff des Anspruchs 1, wie sie aus der US 5 160 314 A bekannt geworden ist, ist eine einzige Ausnehmung zum Durchtritt des Daumens im elastischen Textilteil vorgesehen.

Diese bekannte Handgelenksbandage erfordert je nach Anwendung an der linken oder rechten Hand eines Benutzers einen gesonderten Schnitt und eine entsprechende Anordnung der Verschlussriemen sowie ggf. die jeweils handinnenseitige Anordnung der die Fixationschiene aufnehmenden Tasche. Darüber hinaus sind diese bekannten Handgelenksbandagen in unterschiedlichen Größen insbesondere wegen des unterschiedlichen Hand- bzw. Unterarmumfanges der in Frage kommenden Benutzer vorrätig zu halten.

Aufgabe der vorliegenden Erfindung ist es, eine Handgelenksbandage der eingangs genannten Art zu schaffen, die ohne Passformeinbußen sowohl an der linken als auch an der rechten Hand eines Benutzers getragen werden kann.

Zur Lösung dieser Aufgabe sind bei einer Handgelenksbandage der eingangs genannten Art die im Anspruch 1 angegebenen Merkmale vorgesehen.

Durch die erfindungsgemäßen Maßnahmen ist erreicht, dass die Handgelenksbandage durch Drehen um eine Achse senkrecht zu ihrer Längsrichtung entweder an der linken oder an der rechten Hand getragen werden kann. Dabei bleiben Innen- und Außenseite der Handgelenksbandage erhalten, während lediglich die beiden Längsendbereiche der Handgelenksbandage von der Handinnenfläche zur Unterarminnenseite und umgekehrt wechseln. Das Verschließen bzw. Aneinanderbringen der freien Enden des Textilteils erfolgt in jedem Falle an den dem Daumen abgewandten Kanten von Mittelhand und Unterarm bzw. Handgelenk. Außerdem können dadurch mehrere Konfektionsgrößen zusammengefasst werden.

Gemäß einem bevorzugten Ausführungsbeispiel ist nach den Merkmalen des Anspruchs 2 in einem Zwischenbereich des Textilteils eine einzige längsgerichtete durchgehende Ausnehmung vorgesehen, deren Längsendbereiche die jeweilige Daumenausnehmung bilden.

Eine bevorzugte Ausgestaltung nach den Merkmalen des Anspruchs 3 ergibt eine einfachere Herstellung und einen einfacheren Zuschnitt des Textilteils. Außerdem wird dadurch die Passform sowohl für linksseitiges als auch für rechtsseitiges Tragen gewährleistet.

Mit den Merkmalen nach Anspruch 4 ist ein verbesserter Sitz des Textilteils unterhalb des Daumengelenks im Bereich des Handgelenks erreicht.

Die Möglichkeit, sogenannte Fixationschiene an der Innenseite der Handgelenksbandage vorzusehen, ist bei den bekannten Handgelenksbandagen bereits vorhanden. Teilweise sind derartige Fixationschiene im und zum Bereich der Handinnenfläche um ihre Längsachse winklig abgebogen. Um auch bei solchen abgebogenen Fixationschienen eine Linksund Rechtsverwendbarkeit zu gewährleisten, sind die Merkmale nach Anspruch 5 vorgesehen.

Weitere Ausgestaltungen für eine verbesserte Aussteifung des Handgelenks ergeben sich durch die Merkmale des Anspruchs 6 und/oder 7.

Für einen einfachen Wechsel und gegen ein unbeabsichtigtes Herausrutschen von Fixationsschiene und/oder starrer Schiene aus der Tasche bzw. Aufnahme sind zweckmäßigerweise die Merkmale nach Anspruch 8 vorgesehen.

Mit den Merkmalen nach Anspruch 9 und/oder 10 und/oder 11 ist eine wirkungsvolle und einfache Handhabung des Verschlusses der Handgelenksbandage erreicht. Auch aufgrund dieser Ausgestaltung ist es darüber hinaus ohne Passformeinbussen möglich, mit einer einzigen Größe oder allenfalls zwei Größen an Handgelenksbandagen zur Anpassung an unterschiedliche Handabmessungen auszukommen.

Ein weiterer Vorteil, der sich aus der Kombination mehrerer Merkmale der einzelnen Ansprüche ergibt, besteht darin, dass die textile Fläche des Textilteils auf ein Minimum begrenzt ist, ohne dass darunter die Passform bzw. die medizinische und therapeutische Wirkung einer derartigen Handgelenksbandage leidet.

Weitere Einzelheiten der Erfindung sind der folgenden Beschreibung zu entnehmen, in der die Erfindung anhand des in der Zeichnung dargestellten Ausführungsbeispieles näher beschrieben und erläutert ist. Es zeigen:
- Figur 1: in perspektivischer schematischer Darstellung eine Handgelenksbandage gemäß einem bevorzugten Ausführungsbeispiel vorliegender Erfindung im Tragezustand an einer rechten Hand,
- Figur 2: in perspektivischer Darstellung die Handgelenksbandage nach Figur 1 ohne Hand und im Zustand zum Ein- bzw. Anschlaufen der rechten Hand sowie in Umfangsrichtung um 90° gedreht,
- Figur 3: in Außenansicht den Zuschnitt der ausgelegten Handgelenksbandage und
- Figur 4: in perspektivischer Darstellung einen Fixationschiene für die Handgelenksbandage.

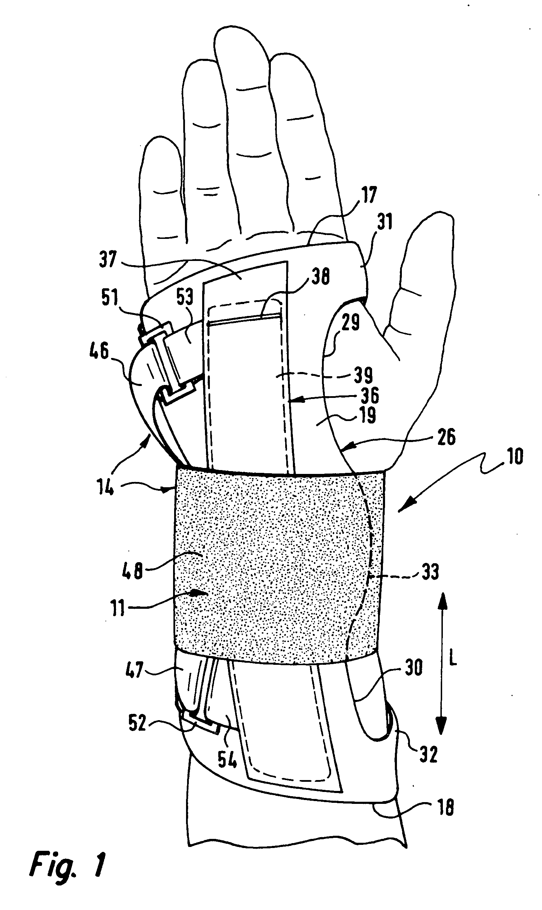

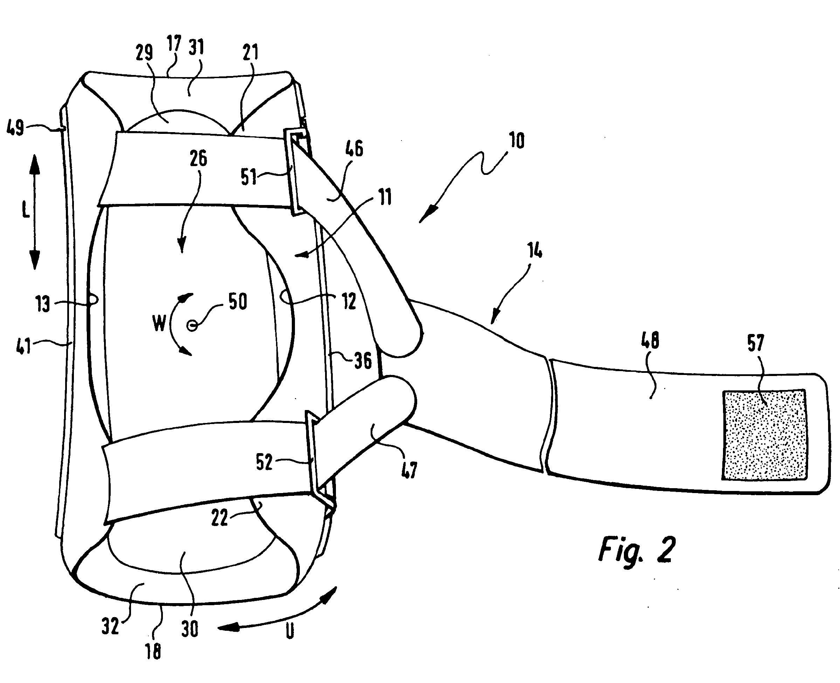

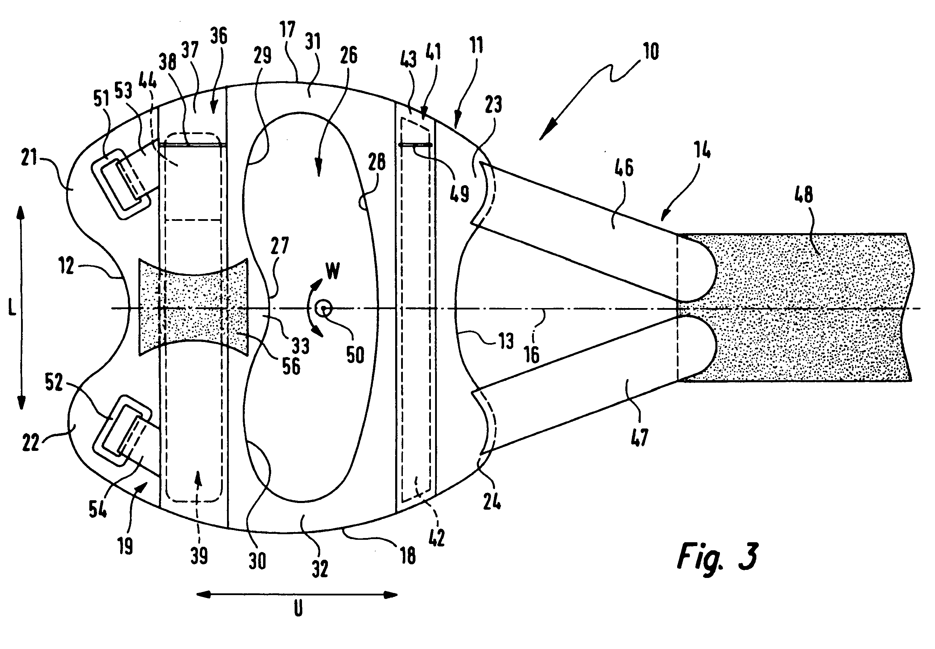

Die in der Zeichnung gemäß einem bevorzugten Ausführungsbeispiel dargestellte Handgelenksbandage 10 besitzt ein in Umfangsrichtung (Doppelpfeil U in Fig. 3) elastisches Textilteil bspw. Gestrickteil 11, das sich im Tragezustand (Figuren 1 und 2) in Längsrichtung L über den Bereich der Mittelhand, des Handgelenks und einem Unterarmbereich erstreckt und umfangsseitig um diese Bereiche gebracht werden kann und dessen freie Enden 12 und 13 mittels eines Verschlusses 14 zu einem strammen Sitz aneinander gezogen und miteinander verbunden werden.

Das Textilteil 11 ist ein in Umfangsrichtung U elastisches und biegeelastisches textiles Gewebe, das senkrecht zur Umfangsrichtung in Längserstreckung L nicht dehnbar ist, also solcher Art, wie es bei üblichen Handgelenksbandagen Verwendung findet.

Das Textilteil 11 und der Verschluss 14 sind zu einer gedachten sich in Umfangsrichtung U erstreckenden Querachse 16 symmetrisch (Figur 3). Die in Umfangsrichtung U verlaufenden oberen und unteren Querränder 17, 18 des Textilteils 11 sind nach außen gewölbt geformt. Die in Längsrichtung L verlaufenden freien Enden 12 und 13 sind zu den oberen und unteren Querrändern 17, 18 angrenzend mit in Umfangsrichtung vorstehenden Lappen 21, 22 bzw. 23, 24 versehen, wobei die Lappen 21 und 22 etwas länger als die Lappen 23 und 24 ausgebildet sind. In einem bezüglich der Umfangsrichtung außermittigen Bereich ist eine sich über einen weiten Teil der Länge des Textilteils 11 erstreckende, in Ansicht etwa oval ausgebildete Ausnehmung 26 vorgesehen, die an einer Längsseite etwa mittig mit einer Einbuchtung 27 versehen ist. Die konkave Einbuchtung 27 in der Ausnehmung 26 weist zum freien Ende 12 hin, während der durchgehend konvexe Randbereich 28 der Ausnehmung 26 dem freien Ende 13 zugewandt ist. Die Ausnehmung 26 lässt sich in einen dem oberen Querrand 17 zugewandten Ausnehmungsteil 29 und in einen dem unteren Querrand 18 zugewandten Ausnehmungsteil 30 unterteilen. Die beiden Ausnehmungsteile 29, 30 sind, den jeweiligen Querrändern 17, 18 zugewandt, von einer schmalen Lasche 31 bzw. 32 begrenzt, die Teil des Textilteils 11 sind. Die beiden Ausnehmungsteile 29 und 30 dienen jeweils zum Durchtritt des Daumens und Daumengelenkteils einer linken bzw. rechten Hand eines Benutzers, während die schmalen Laschen 31 und 32 dabei jeweils zwischen Daumen und Zeigefinger vom Handrücken zur Handinnenseite der linken bzw. rechten Hand verlaufen. Ein die Einbuchtung 27 in der Ausnehmung 26 bildender und die beiden Ausnehmungsteile 29, 30 optisch voneinander trennender Vorsprung 33 befindet sich im Tragezustand unterhalb des Daumengelenks.

Auf der gemäß Figur 3 dargestellten Außenseite 19 des Textilteils 11 ist zwischen der Ausnehmung 26 und dem freien Ende 12 eine längsverlaufende Tasche 36 vorgesehen, die durch eine streifenartige Stofflage 37, die auf das Textilteil 11 aufgenäht ist, im Wesentlichen über die gesamte Längserstreckung L des Textilteils 11 gebildet ist. Die Tasche 36 ist über einen dem oberen Querrand 17 zugewandten Schlitz 38 in der oberen Stofflage 37 zugängig. In die Tasche 36 ist eine Fixationschiene 39 auswechselbar einschiebbar. Der Schlitz 38 ist nahe einem Endbereich vorgesehen, so dass die Tasche 36 in einem sehr langen Hauptaufnahmebereich und in einem sehr kurzen Einaufnahmebereich unterteilt ist. Somit ist der Schlitz 38 so angebracht, dass die Fixationsschiene wie ein Knopfloch eingeschoben werden kann, wobei das Wiederherausrutschen der Schiene dadurch verhindert wird, dass das der Knopflochöffnung näherliegende Ende Richtung Endbereich der Tasche weg vom Knopfloch geschoben wird.

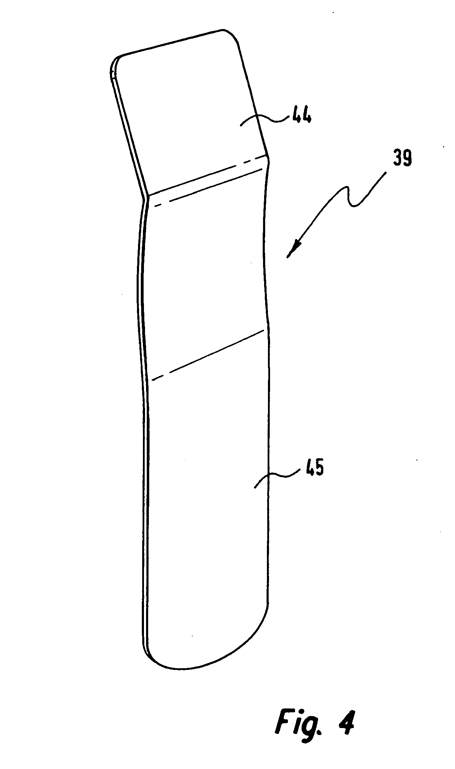

Gemäß Figur 4 ist die Fixationschiene 39 an seinem einen Endbereich 44, der etwa ein Viertel bis ein Drittel der Gesamtlänge ausmacht, zu seine Längsachse um einen spitzen Winkel abgebogen. Die Fixationschiene 39 liegt im Tragezustand mit ihrem abgebogenen Bereich 44 in der Handinnenfläche und mit ihrem anderen in Querrichtung leicht gewölbten Bereich 45 auf den Bereichen von Handgelenk und Unterarm.

Auf der gegenüberliegenden Seite der Ausnehmung 26 befindet sich zwischen dieser und dem freien Ende 13 eine ebenfalls über einen Schlitz 49 offene Aufnahme 41 für eine starre Schiene 42, die über ihre gesamte Längserstreckung eben ist, die sich im Wesentlichen über die gesamte Länge der Aufnahme 41 und damit des Textilteils 11 erstreckt. Die Aufnahme 41 ist außenseitig durch eine relativ schmalstreifige, allseitig vernähte Materiallage 43 auf dem Textilteil 11 begrenzt. Das Auswechseln der Schiene 42 und die Einteilung der Aufnahme 41 kann so wie zur Schiene 39 und Tasche 36 beschrieben sein. Die starre Schiene 42 kann aber auch nicht auswechselbar gehalten sein.

Die Materialauflage 37 bzw. 43 zur Bildung von Tasche 36 bzw. Aufnahme 41 ist in Längserstreckung nicht dehnbar, so dass eine evtl. Elastizität des Textilteils 11 in Längsrichtung unterbunden ist.

Der Verschluss 14 für die Handgelenksbandage 10 besitzt zwei Verbindungsriemen 46 und 47, die mit ihrem einen Ende an den Lappen 23 und 24 des freien Endes 13 derart angenäht sind, dass sie miteinander ein spitzwinkliges Dreieck durch ihre beiden aufeinander zulaufenden anderen Enden bilden. Diese beiden, den Lappen 23 und 24 abgewandten freien Enden sind mit einem etwa die doppelte Breite dieser beiden Verbindungsriemen 46 und 47 aufweisenden Verschlussgurt 48 verbunden, vorzugsweise vernäht. Bevor diese feste Verbindung bzw. Vernähung jedoch stattfinden kann, sind die Verbindungsriemen 46 und 47 durch Ösen 51 bzw. 52 gefädelt, die über Riementeile 53, 54 an der Außenseite des Textilteils 11 im Bereich des dem freien Ende 12 zugewandten Randes der Tasche 36 befestigt vorzugsweise angenäht sind und somit auf den Lappen 21 und 22 liegen. Nach dem Einfädeln der Verbindungsriemen 46 und 47 in die Ösen 51, 52 werden die ersteren mit dem Verbindungsgurt 48 fest verbunden. Der Verbindungsgurt 48 besitzt eine ausreichende Länge, damit die angelegte Handgelenksbandage 10 durch mehrlagiges Umwickeln des Gurtes 48 fest gehalten werden kann (Figur 2). Zur Halterung des Verbindungsgurts 48 ist ein Klettverschlusssystem vorgesehen. Hierzu ist die Tasche 36 außenseitig und längsmittig mit einem Klettverschlussteil 56 bestückt, an dem der innere Bereich des Verschlussgurtes 48 anhaften kann. Der Verschlussgurt 48 besitzt außerdem an seinem Ende außenseitig ebenfalls ein Klettteil 57, damit der Verschlussgurt 48 bei vollständiger Umwicklung mit sich selbst verbunden bzw. an sich selbst haften kann. Erwähnt sei noch, dass der Verschlussgurt 48 in der Querachse 16 verläuft und der gesamte Verschluss 14 symmetrisch zu dieser ist.

Aufgrund der zur Querachse 16 symmetrischen Ausgestaltung der gesamten (mit Ausnahme der Schlitze 38, 49) Handgelenksbandage 10 ist es möglich, diese sowohl für die rechte (Figur 1) als auch für die linke Hand eines Benutzers zu verwenden. Dies bedeutet, dass entweder die gemäß Figur 2 bzw. 3 obere Verbindungslasche 31 oder die gemäß Figur 3 untere Verbindungslasche 32 in dem Bereich zwischen Daumen und Zeigefinger einerseits der rechten Hand und andererseits der linken Hand zu liegen kommt. Dies wird dadurch erreicht, dass die Handgelenksbandage 10 um eine Achse 50 senkrecht zur Querachse 16 gemäß Doppelpfeil W um 180° in die eine oder andere Richtung gewendet wird, was bedeutet, dass der obere Querrand 17 nicht mehr im Bereich der Handaußen- bzw. -innenfläche liegt, sondern sich um den Unterarmbereich legt. Entsprechendes gilt für den gemäß Zeichnung unteren Querrand 18 in umgekehrter Weise. Für Figur 2 heißt dies, dass bei auf dem Kopf stehender Figur 2 die linke Hand mit dem Daumen noch oben und der Handinnenfläche zum Verbindungsgurt 48 gewandt eingeschlauft wird.

Ist eine in Figur 4 dargestellter Fixationschiene 39 vorgesehen, wird dieser aus der Tasche 36 durch den offenen Schlitz 38 herausgezogen und um 180° gedreht in umgekehrter Weise wieder eingeschoben. Die Lage der Fixationschiene 39 ist dann stets derart, dass ihr abgebogenes Ende 44 in der Tasche 36 so angeordnet ist, dass es in an der betreffenden Hand angelegtem Zustand in der Innenfläche der betreffenden (linken oder rechten) Hand liegt.

## Patentansprüche

1. Handgelenksbandage (10), mit einem die Handinnen- und -außenseite sowie das Handgelenk und einen Längenbereich des Unterarms umgebenden an einer Längsseite offenen, in Umfangsrichtung elastischen Textilteil (11), mit einem das Textilteil (11) in Umfangsrichtung zusammenziehbaren Verschluss (14) zwischen den freien Enden (12, 13) des Textilteils (11), mit einer Ausnehmung (26) im Textilteil für den Daumen der Hand in angelegtem Zustand und ggf. mit einer längsverlaufenden in den mittigen Bereich der Handinnenfläche ragenden und eine Fixationschiene (39) aufnehmenden Tasche (36) am Textilteil (11), wobei die Ausnehmung (26) zum Durchtritt des Daumens zwischen den mit dem Verschluss (14) versehenen freien Enden (12, 13) des Textilteils (11) vorgesehen ist, **dadurch gekennzeichnet, dass** die Ausnehmung (26) an beiden Längsendbereichen des Textilteils (11) mit einem Ausnehmungsteil (29, 30) versehen ist.

2. Handgelenksbandage nach Anspruch 1, **dadurch gekennzeichnet, dass** die beiden längsendbereichsseitigen Ausnehmungsteile (29, 30) der Daumenausnehmung (26) ineinander übergehen.

3. Handgelenksbandage nach Anspruch 1 oder 2, **dadurch gekennzeichnet, dass** das Textilteil (11) zu einer mittigen Umfangs- bzw. Querachse (16) symmetrisch ist.

4. Handgelenksbandage nach Anspruch 2 oder 3, **dadurch gekennzeichnet, dass** zwischen den beiden Ausnehmungsteilen (29, 30) das Textilteil (11) mit einem unterhalb des Daumengelenks liegenden Vorsprung (33) versehen ist.

5. Handgelenksbandage nach mindestens einem der vorhergehenden Ansprüche, **dadurch gekennzeichnet, dass** die eine endseitig aus seiner Längsachse zur Handinnenfläche abgebogenen Fixationschiene (39) aufnehmende Tasche (36) an einem Endbereich offen ist.

6. Handgelenksbandage nach mindestens einem der vorhergehenden Ansprüche, **dadurch gekennzeichnet, dass** der Fixationschiene (39) etwa diametral gegenüberliegend eine längsverlaufende starre Schiene (42) am Textilteil (11) gehalten ist.

7. Handgelenksbandage nach Anspruch 6, **dadurch gekennzeichnet, dass** die starre Schiene (42) über die gesamte Länge des Textilteils (11) verlaufend in einer mit einem offenen Endbereich versehenen Aufnahme (41) gehalten ist.

8. Handgelenksbandage nach Anspruch 5 oder 7, **dadurch gekennzeichnet, dass** die Öffnungsschlitze (38, 49) für die Tasche (36) bzw. Aufnahme (41) in einen Hauptaufnahmebereich und einen sehr kurzen Endaufnahmebereich unterteilt.

9. Handgelenksbandage nach mindestens einem der vorhergehenden Ansprüche, **dadurch gekennzeichnet, dass** die freien Enden (12, 13) des Textilteils (11) an zwei einander abgewandten Längsendbereichen mit in Umfangsrichtung vorstehenden Lappen (21 bis 24) versehen sind, über die der verstellbare Verschluss (14) verläuft.

10. Handgelenksbandage nach Anspruch 9, **dadurch gekennzeichnet, dass** an einem Paar Lappen (23, 24) des einen freien Endes (13) des Textilteils (11) Verbindungsriemen (46, 47) des Verschlusses (14) befestigt sind, die dreieckförmig durch eine Öse (51, 52) am anderen freien Ende (12) gefädelt zusammenlaufen und mit einem breiten wickelbaren Verschlussgurt (48) verbunden sind.

11. Handgelenksbandage nach mindestens einem der vorhergehenden Ansprüche, **dadurch gekennzeichnet, dass** der Verschluss (14) zur mittigen Umfangs- bzw. Querachse (16) symmetrisch ausgebildet ist.

## Claims

1. A wrist bandage (10), having a textile part (11) that is elastic in the circumferential direction and open on one longitudinal side and that surrounds the interior and exterior sides of the hand as well as the wrist and a longitudinal area of the forearm, having between the free ends (12, 13) of the textile part (11) a fastener (14) that pulls the textile part (11) together in the circumferential direction, having a cutout (26) in the textile part for the thumb of the hand in the bound state and, if necessary, having a longitudinal pocket (36) on the textile part (11) that accommodates an immobilizing splint (39) and that extends into the central area of the interior surface of the hand, whereby the cutout (26) is provided to allow the passage of the thumb between the free ends (12, 13) of the textile part (11) that are furnished with the fastener (14), wherein the cutout (26) is provided with a cutout part (29, 30) on both longitudinal end areas of the textile part (11).

2. The wrist bandage as recited in Claim 1, wherein the two cutout parts (29, 30) of the thumb cutout (26) in the longitudinal end areas merge into each other.

3. The wrist bandage as recited in Claim 1 or 2, wherein the textile part (11) is symmetrical to a central circumferential and transverse axis (16).

4. The wrist bandage as recited in Claim 2 or 3, wherein between the two cutout parts (29, 30) the textile part (11) is provided with a projection (33) that is situated beneath the thumb joint.

5. The wrist bandage as recited in at least one of the preceding claims, wherein the pocket (36) that on the end side accommodates an immobilizing splint (39) that is bent from its longitudinal axis to mold to the interior surface of the hand is open in one end area.

6. The wrist bandage as recited in at least one of the preceding claims, wherein a longitudinal rigid bar (42) is held on the textile part (11) roughly diametrically opposite the immobilizing splint (39).

7. The wrist bandage as recited in Claim 6, wherein the rigid bar (42) is held over the entire length of the textile part (11) in a receptacle (41) that is provided with an open end area.

8. The wrist bandage as recited in Claim 5 or 7, wherein the opening slots (38, 49) for the pocket (36) and the receptacle (41) are subdivided into a main receiving area and a very short end receiving area.

9. The wrist bandage as recited in at least one of the preceding claims, wherein the free ends (12, 13) of the textile part (11) in two longitudinal end areas facing away from each other are provided with projecting tabs (21 to 24) that protrude in the circumferential direction, over which the adjustable fastener (14) runs.

10. The wrist bandage as recited in Claim 9, wherein on one pair of tabs (23, 24) of the one free end (13) of the textile part (11) connecting straps (46, 47) of the fastener (14) are attached, which run together in triangular fashion, being threaded through an eye (51, 52) on the other free end (12), and which are joined to a wide fastener belt (48) that can be wound.

11. The wrist bandage as recited in at least one of the preceding claims, wherein the fastener (14) is configured so as to be symmetrical to the central circumferential and transverse axis (16).

## Revendications

1. Bandage pour poignet (10), avec une partie textile (11) élastique dans la direction circonférentielle, ouvert sur un côté longitudinal et entourant la paume et le dos de la main, ainsi que le poignet et une partie longitudinale de l'avant-bras, avec une fermeture (14), qui est disposée entre les extrémités libres (12, 13) de la partie textile (11) et qui est propre à resserrer la partie textile (11) dans la direction circonférentielle, avec un évidement (26) pour le pouce dans la partie textile dans la position montée, et, le cas échéant, avec une poche (36) sur la partie textile (11), laquelle est orientée dans la direction longitudinale en s'avançant dans la zone centrale de la paume de la main et laquelle reçoit un rail de fixation (39), ledit évidement (26) pour le passage du pouce étant prévu entre les extrémités libres (12, 13), munies de la fermeture (14), de la partie textile (11), **caractérisé en ce que** ledit évidement (26) est muni d'une partie (29, 30) dans les deux zones d'extrémité longitudinales de la partie textile (11).

2. Bandage pour poignet selon la revendication 1, **caractérisé en ce que** les deux parties (29, 30) dans les zones d'extrémité longitudinales de l'évidement (26) pour le pouce se prolongent l'une dans l'autre.

3. Bandage pour poignet selon la revendication 1 ou 2, **caractérisé en ce que** la partie textile (11) est symétrique par rapport à un axe périphérique ou transversal (16) médian.

4. Bandage pour poignet selon la revendication 2 ou 3, **caractérisé en ce qu'**entre les deux parties (29, 30) de l'évidement, la partie textile (11) est munie d'une saillie (33) située en dessous de l'articulation du pouce.

5. Bandage pour poignet selon au moins l'une des revendications précédentes, **caractérisé en ce que** la poche (36), recevant un rail de fixation (39) cintré à son extrémité de manière à sortir de son axe longitudinal vers la paume de la main, est ouverte au niveau d'une zone d'extrémité.

6. Bandage pour poignet selon au moins l'une des revendications précédentes, **caractérisé en ce que** le rail de fixation (39) est maintenu sur la partie textile (11) en étant sensiblement diamétralement opposé à un rail (42) rigide orienté dans le sens longitudinal.

7. Bandage pour poignet selon la revendication 6, **caractérisé en ce que** le rail (42) rigide, s'étendant sur toute la longueur de la partie textile (11), est maintenu dans un logement (41) muni d'une zone d'extrémité ouverte.

8. Bandage pour poignet selon la revendication 5 ou 7, **caractérisé en ce que** la fente d'ouverture (38, 49) respectivement pour la poche (36) et le logement (41) divise ceux-ci en une zone de réception principale et en une très courte zone de réception d'extrémité.

9. Bandage pour poignet selon au moins l'une des revendications précédentes, **caractérisé en ce que** les extrémités libres (12, 13) de la partie textile (11) sont munies, au niveau de deux zones d'extrémité longitudinales opposées l'une à l'autre, de languettes (21 à 24), qui s'avancent en saillie dans la direction circonférentielle et sur lesquelles passe la fermeture (14) réglable.

10. Bandage pour poignet selon la revendication 9, **caractérisé en ce qu'**au niveau d'une paire de languettes (23, 24) de l'une des extrémités libres (13) de la partie textile (11) sont fixées des lanières de jonction (46, 47) de la fermeture (14), lesquelles convergent en triangle et s'enfilent dans l'autre extrémité libre (12) à travers un oeillet (51, 52) et lesquelles sont reliées à une large sangle de fermeture (48) enroulable.

11. Bandage pour poignet selon au moins l'une des revendications précédentes, **caractérisé en ce que** la fermeture (14) est réalisée symétriquement par rapport à l'axe périphérique et transversal (16).
